# EUROPEAN PATENT SPECIFICATION

(11) **EP 0 975 337 B1**
(45) Date of publication and mention of the grant of the patent: **14.05.2003**
(21) Application number: 98924087.4
(22) Date of filing: 20.03.1998
(51) Int. Cl.: A61K 9/20, A61K 31/19

(54) **FAST RELEASE COMPRESSED TABLET OF FLURBIPROFEN**
SCHNELLFREISETZENDE KOMPRIMIERTE FLURBIPROFEN ENTHALTENDE TABLETTE
TABLETTES DE FLURBIPROFEN A LIBERATION RAPIDE

(30) Priority: 22.03.1997 GB 9705989
(43) Date of publication of application: 02.02.2000
(73) Proprietor: The Boots Company PLC, Nottingham, Nottinghamshire NG2 3AA (GB)
(72) Inventor: JONES, Huw, Lyn, The Boots Company plc, Nottingham NG2 3AA (GB); BUTLER, Malcolm, Richard, Wollaton, Nottingham NG8 2PG (GB)
(74) Representative: Frith, Richard William
(86) International application number: EP9801831
(87) International publication number: WO98042310

(56) References cited:
- EP-A- 0 636 364
- WO-A-92/04018
- WO-A-93/23026
- US-A- 3 973 037
- US-A- 4 844 907
- PATENT ABSTRACTS OF JAPAN vol. 097, no. 007, 31 July 1997 & JP 09 071523 A (RIYUUKAKUSAN:KK), 18 March 1997 & DATABASE WPI Section Ch, Week 9721 Derwent Publications Ltd., London, GB; Class A96, AN 97-231144 & JP 09 071523 A (RYUKAKUSAN KK) , 18 March 1997

## Description

The present invention relates to compressed pharmaceutical compositions containing flurbiprofen, to processes to prepare said compositions and to therapeutic uses thereof.

Flurbiprofen is the name given to the compound 2-(2-fluoro-4-biphenylyl)propionic acid. Flurbiprofen has a chiral centre and can exist in two enantiomeric forms, namely S(+)-flurbiprofen or R(-)-flurbiprofen, or as a mixture thereof. Flurbiprofen is normally supplied as a racemic mixture (1:1) of these enantiomers, although S(+)- and R(-)-flurbiprofen have individually been proposed for therapeutic use. Flurbiprofen is a member of the class of compounds known as non-steroidal anti-inflammatory agents and thus it is primarily used for the treatment of pain, inflammation and fever. It is currently available in the UK as a prescription drug under the trade name Froben (RTM) indicated for mild to moderate pain, headaches, dysmenorrhoea, post-operative pain, gout, back pain, pyrexia, rheumatoid disease, osteoarthritis, ankylosing spondylitis and acute muscoskeletal disorders, generally at doses up to 300 mg per day. The same formulation is also available in other countries, for example in France under the trade name Cebutid (RTM).

It is desired to formulate a tablet containing flurbiprofen for oral administration to a patient. The tablet is primarily intended to treat painful conditions but is also for use in the other known indications of flurbiprofen. It is desired that the tablet composition is able to deliver up the drug for absorption as quickly as possible. However, flurbiprofen is defined as being a practically insoluble drug. It is also a drug with an acidic group. These factors need to be considered to provide a fast-acting tablet. In addition, relatively fast disintegration of the tablet, facilitating desirable dissolution properties, are particularly valuable characteristics for a therapeutic composition adapted for the treatment of pain where fast onset of action is desirable. Furthermore, it is important that any tablet produced is sufficiently robust at a range of temperatures and humidities that it does not break up during the tablet manufacturing process, for example during any film coating stage, or during packaging andlor transit to a desired location. It is thus desired to prepare a flurbiprofen tablet which has satisfactory hardness properties (as demonstrated by its crushing strength) but also advantageous disintegration and dissolution characteristics. It is also important that the tablet can be manufactured by a relatively inexpensive and facile process and that it is stable on storage.

It is of key importance to a large scale manufacturing process that the specification of the tablets produced, eg tablet weight, disintegration time, does not change significantly at any point during the production process. Thus, in any large scale tablet production process, it is important to continuously monitor the characteristics of the tablets being produced. During the production run, tests are regularly made to confirm that measures such as disintegration time, tablet hardness and tablet weight are all maintained within a tightly defined specification. These and other characteristics may be caused to vary by factors such as the particle size of the ingredients fed into the dies of the tabletting machine or the humidity conditions encountered during the blending and tabletting process. Appropriate steps have to be taken within the manufacturing process to keep the tablets produced within the desired specification.

As an example of variability, granules of flurbiprofen with one or more excipients may be prepared by a conventional granulating process, dried and then sieved to remove the coarser particles. However, there still remains a range of particle sizes varying from fine material up to the largest particle size allowed by the sieve. Although steps are taken to ensure that the same blend of particles is fed into each die of the tabletting machine, at some times it is found that one batch of material fed into the dies has a smaller median particle size than another batch, or that material within the same batch fed into the dies at one point is different to that fed into the dies at another point of the process. Variations such as this are likely to make very small changes in the tablet weight, hardness and disintegration properties etc of the tablets produced from such batches. Thus, in order to keep the tablets within their specification, slight adjustments in the tabletting process may be necessary. One means of ensuring this is to increase or decrease the compaction pressure of the tabletting machine.

Previously, however, it has been found that a slight increase in the tabletting compaction pressure for a flurbiprofen tablet may lead to a significant increase in the disintegration time of the resulting tablet. It is thus important to produce a flurbiprofen composition which shows fairly constant properties over a range of compaction pressures so that the compaction pressure can be raised or lowered as desired to keep the resulting tablet within a tightly defined specification.

It is known to combine flurbiprofen with sugar materials and/or starch materials as a compressible carrier (in particular lactose and maize starch (or corn starch)) in tabletted formulations. Particular examples of flurbiprofen tablets have already been disclosed in the art. For example, US patent 4443476, European patent application 068838A, GB patent application 2111493A and WO 92/04018 all disclose specific examples of combining flurbiprofen or a salt or enantiomers thereof with lactose and maize starch (or corn starch) as a compressible carrier in a tabletted formulation.

An improved flurbiprofen tablet is desired which particularly combines satisfactory hardness properties with rapid disintegration properties so that it is particularly adapted to treat painful conditions as quickly as possible.

It has been proposed to mix non-steroidal anti-inflammatory drugs such as ibuprofen with a wide variety of excipients. For example, WO 89/02266 discloses forming a granule for tabletting compressing 50-85% w/w ibuprofen, 1-5% w/w cellulose disintegrant of an internally cross-linked alkali (sodium) carboxymethylcellulose, 10-25% w/w pre-gelatinised starch and 6-18% w/w microcrystalline cellulose. However, the technical teaching regarding the tabletting of the high dose drug ibuprofen cannot be applied to the present case where the unit dosages are generally much smaller.

In addition, WO 92/04018 discloses generally that solid forms of administration comprising the flurbiprofen enantiomers or the flurbiprofen pseudoracemate (equal quantities of previously separated R(-) and S(+)-flurbiprofen enantiomers) may contain 20-80% of filling materials including inter alia, starch, lactose, glucose, mannitol, calcium carbonate, calcium phosphate, cellulose and the like. A disintegration agent may also be added in an amount of 2-10% including carboxymethyl starch, carboxymethyl cellulose, polyvinylpyrrolidone and silica gel. It is disclosed that the powders may be mixed dry and subsequently moist granulated with a binding agent prior to compression into tablets. Examples of tablet compositions are given in which the flurbiprofen pseudoracemate is combined with a compressible carrier comprising either a mixture of lactose and maize starch (the compressible carrier being present to an extent greater than 50% of the formulation) or microcrystalline cellulose and gelatine (the compressible carrier forming less than 25% by weight of the mixture). However, this provides no teaching that enhanced crushing strengths and disintegration times of flurbiprofen tablets can be achieved by the particular combination of compressible components in accordance with the present invention.

We have now found that by incorporating flurbiprofen or a salt thereof in an amount of 1-50% w/w of the composition with a particularly selected mixture of materials (as defined herein) as the compressible carrier material, we have been able to formulate a robust tablet having good disintegration properties over a range of compaction pressures and which is capable of large scale production allowing for flexibility in the tablet production process.

Accordingly, the present invention provides a fast-release homogeneous compressed tablet composition comprising:-
i) 1-50% by weight flurbiprofen or a pharmaceutically acceptable salt thereof; and
ii) 50-99% by weight compressible carrier material comprising a disintegrant and a compressible component selected from a sugar component, a starch component and an alkaline earth metal component;
characterised in that the compressible carrier material further comprises microcrystalline cellulose present in a ratio to said compressible component of 1:4 to 4:1 parts by weight;
and further characterised in that the crushing strength of the tablet as measured by a Schleuniger hardness meter is in the range 5-15 kgf and that the disintegration time measured according to the European Pharmacopoeia, 1986, Ref V.5.1.1 (updated 1997) is less than 10 minutes.

Tablets according to the present invention also have advantageous dissolution properties. This can be seen by the time taken for a certain percentage of the flurbiprofen to be dissolved in a buffered aqueous medium under the Dissolution Test set out in USP 23 (1995 Edition) Dissolution Apparatus 2 (p690). The time taken for 50% (T₅₀) and 80% (T₈₀) of the flurbiprofen to be dissolved are indications of valuable dissolution properties. We prefer to measure the % of drug dissolved in the dissolution medium after fixed time points, eg after any of 10, 20, 30, 45 and 60 minutes, which also indicate valuable dissolution properties.

Microcrystalline cellulose is a crystalline material known for its compressibility and disintegration characteristics. However, it has not previously been proposed that when combined with a compressible component as defined above, a flurbiprofen tablet having such valuable properties would be produced. In particular a desirable crushing strength can be maintained over a wider range of compression pressures than could be expected in the absence of the mixture.

A particularly preferred feature of the invention is that the preferred tablet compositions maintain a disintegration time of less than 10 minutes over the whole range of crushing strength values in the range 5-15 kgf. The composition according to the present invention is also sufficiently flexible that the same excipient proportions can cater for a variety of flurbiprofen dosages. For example, if a higher dose is required in comparison to any given dose this can be achieved in two or more ways. In one method, all the ingredients of the given dose may be multiplied up as required to give the larger dose, eg twice as much ingredients are fed to the dies to increase the dose from 6.25 mg to 12.5 mg which will provide a tablet of twice the weight. In another method, the overall tablet weight can be maintained at the same value and the amount of compressible carrier material reduced to cater for the increase in drug content whilst maintaining the proportions of microcrystalline cellulose to compressible component at substantially the same ratio. It is a further advantage that tablets of different shapes with acceptable dissolution properties can be manufactured.

As well as having valuable crushing strength, disintegration and dissolution properties, the tabletted composition also satisfies the fundamental tabletting requirement of not sticking to the tablet punches. Also, it is not subjected to capping (removal of one side of the tablet after compaction). The tablets may be made by a relatively facile process of combining all the ingredients and compressing into tablets. It does not require particular treatments of any of the ingredients or keeping certain materials in separate layers. It is of particular advantage that such tablets can be produced on high speed tabletting machines.

The flurbiprofen may be in the form of racemic flurbiprofen, as substantially pure enantiomers, S(+)-flurbiprofen and R(-)-flurbiprofen, or a mixture of the enantiomers. The flurbiprofen may also be present in the form of a pharmaceutically acceptable salt thereof, such as an alkali metal salt, eg sodium or potassium; an alkaline earth metal salt, eg calcium or magnesium; a metal salt, eg aluminium; an amino acid salt, eg lysine or arginine; or an amine salt, eg meglumine. Soluble salts are preferred. Most preferably flurbiprofen or the sodium salt thereof, either in the form of the racemate or as either one of the enantiomers (especially S(+)-flurbiprofen), is employed in a tablet according to the present invention. Most preferably racemic flurbiprofen is used.

The particle size of the flurbiprofen or salt thereof should be such as to facilitate the manufacturing process. Preferably the mean particle size is in the range 5-1000 µm, preferably 50-600 µm. The flurbiprofen may either be present as a crystalline material having this particle size or it may be subjected to a pre-granulation stage with excipients to produce a granule which is capable of free flow and compression having a median particle size in this range.

The flurbiprofen or salt thereof forms the sole active ingredient in the composition and is present to an extent of 1-50% by weight, preferably to an extent of 2-30% by weight, more preferably 2-24% by weight and most preferably 2.5-20% by weight of a composition according to the present invention. Particularly preferred tablet compositions comprise 2-30% by weight flurbiprofen or a soluble salt thereof, especially 2.5-20% by weight racemic flurbiprofen.

Unit dosages may comprise flurbiprofen or salt thereof to provide a flurbiprofen equivalent dose in the range 5-400 mg, eg 5 mg, 6.25 mg, 8.75 mg, 10 mg, 12.5 mg, 15 mg, 20 mg, 25 mg, 50 mg, 100 mg, 200 mg, 300 mg or even 400 mg. The dose of each enantiomer could be expected to be reduced to up to half the dose of racemic flurbiprofen to achieve the desired therapeutic effect. Unit doses may be administered as a single tablet or as several tablets as desired. It is envisaged that the flurbiprofen or salt will be the only active ingredient present as the presence of other active ingredients will affect the properties of the tablet.

The tablet composition comprises 50-99% w/w compressible carrier material, preferably 60-95% w/w and more preferably 70-92% w/w compressible carrier material. The ratio of flurbiprofen or salt thereof to the compressible carrier material is suitably within the range 1:1-1:40 parts by weight, preferably 1:1 to 1:32 and more preferably 1:1 to 1:10 parts by weight.

The compressible carrier material is adapted for combination with flurbiprofen to provide a tablet having sufficient hardness but which has relatively fast disintegration properties which do not vary significantly over a range of compression pressures. The compressible carrier material comprises a mixture of:-
(a) a disintegrant;
(b) microcrystalline cellulose; and
(c) a compressible component selected from a sugar component, a starch component and an alkaline earth metal component, or mixtures thereof, such that the ratio of microcrystalline cellulose to said compressible material is in the range 1:4 to 4:1 parts by weight.

The compressible component may be soluble or insoluble in water. The compressible component may comprise a plurality of materials selected from a sugar component, a starch component or an alkaline earth metal component. Preferably, however, one, two or three materials are chosen from any of these particular classes of materials. Further preferably a single material is selected as the compressible component.

The sugar component may comprise various forms of sugars, including, but not limited to, lactose, sucrose, dextrose, dextrin, cyclodextrin, maltodextrin or sugar alcohols, including mannitol, sorbitol, xylitol, or mixtures thereof. Preferably a single material as listed above is selected as the sugar component, more preferably lactose. Non-limiting examples of the starch component include maize starch, potato starch, corn starch, wheat starch or pre-gelatinised starch. Preferably a single material is selected as the starch component, most preferably maize starch. The alkaline earth metal component may include calcium or magnesium compressible materials including calcium sulphate, calcium carbonate, calcium phosphate compounds such as calcium diphosphate, tricalcium phosphate and dicalcium phosphate as well as magnesium sulphate, magnesium carbonate, magnesium oxide and magnesium hydroxide or mixtures thereof. Other materials will also be known by the person skilled in the art. Preferred are calcium phosphate compounds, especially a single material selected from the phosphate compounds listed above, especially dicalcium phosphate and tricalcium phosphate.

An especially preferred compressible component is lactose.

The ratio of microcrystalline cellulose to said compressible component is in the range 1:4 to 4:1 parts by weight, preferably 4:1 to 1:2 parts by weight, more preferably 2:1 to 1:2 parts by weight, especially 1:1 to 1:2 parts by weight. In particularly preferred compositions the ratio of microcrystalline cellulose to said compressible component is in the range 1:1 to 1:1.5 parts by weight.

According to the present invention a tablet suitably comprises 20-60% w/w microcrystalline cellulose, preferably 20-50% w/w, more preferably 25-40% w/w microcrystalline cellulose. Suitably a tablet comprises 10-60% w/w compressible component as defined herein, preferably 15-50%, more preferably 30-50% w/w.

The compressible carrier material according to the present invention also comprises a tablet disintegrant. Examples of disintegrating components include one or more of cross-linked polyvinylpyrrolidone, starch and its derivatives, sodium starch glycollate, alginic acid, methyl cellulose, hydroxymethyl cellulose, wood cellulose, croscarmellose sodium and magnesium aluminium silicate. Preferably croscarmellose sodium is employed in accordance with the present invention. Such disintegrating agents are suitably used to an extent of 1-12% w/w, preferably 3-10% w/w, more preferably 5-8% w/w of the tablet.

A preferred tablet according to the present invention comprises
i)1-25% by weight flurbiprofen or a pharmaceutically acceptable salt thereof; and
ii) 75-99% by weight compressible carrier material comprising a disintegrant and a sugar component as the compressible component,
characterised in that the compressible carrier material further comprises microcrystalline cellulose present in a ratio to said compressible component of 1:2 to 2:1 parts by weight;
and further characterised in that the crushing strength of the tablet is in the range 5-15 kgf and that the disintegration time is less than 10 minutes.

The compressible carrier material may comprise additional diluent materials well-known to those skilled in the art, including other cellulose materials as appropriate. Preferably, however, the compressible carrier material consists essentially of a mixture of a disintegrant, microcrystalline cellulose and a single compressible component selected from a sugar component, a starch component or a phosphate component, most preferably lactose. Most preferably the compressible carrier material consists essentially of croscarmellose sodium as disintegrant and microcrystalline cellulose together with lactose as the compressible component.

If convenient, the tablet according to the present invention may optionally include up to 20% w/w formulation excipients. Thus it may optionally include a tablet lubricant. Lubricants such as stearic acid, sodium lauryl sulphate, polyethylene glycol, hydrogenated vegetable oil, calcium stearate, sodium stearyl fumarate or magnesium stearate may be employed, to an extent of 0.1-5% w/w, preferably 0.5-2% w/w of the tablet. Additionally the tablets may also include up to 4% by weight of a flow aid such as talc or colloidal silicon dioxide (preferably 0.5-2.0% by weight of the tablet). If the flurbiprofen is pre-granulated before formation into tablets, the tablet composition may also contain formulation excipients useful in the pre-granulation process, such as a binder (eg polyvinylpyrrolidone). Other formulation ingredients as desired may also be used within the knowledge of the person skilled in the art, for example with reference to Remington's Pharmaceutical Sciences.

A preferred tablet consists essentially of the flurbiprofen or salt thereof and the compressible carrier material within the limits defined herein. Further preferably the tablet composition contains no additional components.

A tablet according to the present invention may be coated, eg with a conventional sugar or film coating which has minimal effect on the disintegration time. A preferred tablet of the present invention is film coated, such as by spraying tablets with a solution comprising hydroxypropyl methylcellulose.

A tablet produced in accordance with the present invention may be compressed to have a crushing strength in the range 5-15 kgf, more preferably 6-12 kgf. This can be achieved, for example, using a standard single punch tabletting machine or a rotary tabletting machine having conventional compression forces (eg in the range 100-200 MPa).

It will be appreciated by the person skilled in the art that due to the different excipients used in the tablet and varying amounts thereof, that for any compression pressure, different tablets will exhibit different crushing strength and disintegration times. Preferred dosage forms exhibit a crushing strength of 5-15 kgf and a disintegration time of less than 10 minutes at a compression force above 80 MPa. More preferred compositions exhibit a crushing strength of 5-15 kgf and disintegration time of less than 10 minutes when compressed at a compression force in the range 100-200 MPa, especially 100-150 MPa, such as by a standard tabletting machine, eg a rotary tabletting machine. Such compression pressures include, 110 MPa, 120 MPa and 130 MPa. Especially preferred tablets exhibit a crushing strength of 5-10 kgf and a disintegration time of less than 10 minutes when compressed at all pressures in the range 100-200 MPa.

The disintegration time of the tablet formed in accordance with the present invention is less than 10 minutes. A preferred disintegration time is less than 6 minutes (eg 1-6 minutes), more preferably less than 5 minutes (eg 1-5 minutes).

In preferred tablet compositions according to the present invention the T₅₀ and T₈₀ dissolution times (defined hereinabove) are less than 30 minutes. Preferably the T₈₀ is less than 20 minutes, most preferably less than 10 minutes. Preferably the T₅₀ is less than 20 minutes, more preferably less than 10 minutes and most preferably less than 8 minutes. Typically, in such compositions greater than 70% w/w, more preferably greater than 75% w/w and most preferably greater than 85% of the flurbiprofen or salt thereof is dissolved within 20 minutes, more preferably within 10 minutes.

The tablets formed in accordance with the present invention are prepared by compression. The flurbiprofen or salt thereof is combined with the compressible carrier material and any other optional ingredients added and compressed into a tablet. Tabletting machines typically work at a range of compression forces of 100-200 MPa, including 100-140 MPa. The tablets may either be made by blending a powder form of the ingredients together and then directly compressing the mixture into tablets or by carrying out a pre-granulation stage in which the flurbiprofen is mixed with one or more of the excipients (eg a disintegrant andlor a compressible carrier) and formed into a granule before combining with the other necessary ingredients and compressing into a tablet. It is preferred that the flurbiprofen and compressible carrier material are in intimate admixture in a homogeneous blend of tablet ingredients.

Thus there is provided a process to prepare a fast-release homogeneous compressed tablet composition comprising:-
i) 1-50% by weight flurbiprofen or a pharmaceutically acceptable salt thereof; and
ii) 50-99% by weight compressible carrier material comprising a disintegrant and a compressible component selected from a sugar component, a starch component and an alkaline earth metal component;
characterised in that the compressible carrier material further comprises microcrystalline cellulose present in a ratio to said compressible component of 1:4 to 4:1 parts by weight;
and further characterised in that the crushing strength of the tablet is in the range 5-15 kgf and that the disintegration time is less than 10 minutes
which comprises combining the ingredients defined in (i) and (ii) above, optionally with other tabletting ingredients to form a homogeneous mixture followed by compression into a tablet.

Thus the dosage forms may be formed by sieving the powdered ingredients into a container and then blending all of the ingredients to prepare a homogenous mixture. The mixture may then be fed directly to a tabletting machine for direct compression into tablets.

Additionally, there may be a pre-granulation stage prior to tabletting in which a flurbiprofen granule may be prepared by wet granulation. The flurbiprofen and a portion of or all of the compressible component, optionally including a portion of or all of the disintegrant, is fed into a conventional granulating machine. A granulating liquid containing a solution of a binder, for example, polyvinylpyrrolidone, in a solvent such as industrial methylated spirit or a suitable alcoholic solvent, eg a C₁₋₁₀ alcohol, especially isopropanol, is added to the mixture in the granulating machine. Preferably, the flurbiprofen is formed into a granule solely with the compressible carrier material in the presence of an inert solvent. After blending until the desired median particle size is reached, the granules are dried. The granulated material is sieved to remove the coarser particles and then mixed with other excipients as necessary, including any remaining portion of the compressible excipient and compressed into a tablet according to the present invention.

In therapeutic use the tablets of the present invention are administered orally. Flurbiprofen and its salts are primarily anti-inflammatory, analgesic and anti-pyretic agents. The tablets of the present invention are indicated for use in the treatment of oral therapeutic uses for which flurbiprofen is known to be effective including mild to moderate pain, headache, dysmennhorea, post-operative pain, gout, back pain, pyrexia, rheumatoid disease, osteoarthritis, arthritic disease, ankylosing spondylitis, acute muscoskeletal disorders and for the relief of painful conditions such as migraine, headaches, backaches, dental pain, neuralgia, cancer pain and the pain from sprains and strains. The tablets according to the present invention are usually administered according to the therapeutic treatment desired and will be within the knowledge of any prescribing physician (as evidenced by MIMs) or pharmacist as appropriate or as indicated on a leaflet to the patient. Flurbiprofen may generally be administered up to a daily maximum of 600 mg, although a maximum daily dose of 300mg is more usual eg 5-300 mg. As well as prescription use, non-prescription use is also envisaged. It may suitably be administered in one, two or three doses during the day at appropriate intervals for most indications, but a more frequent dose may be appropriate to treat some conditions.

In a further aspect, the present invention provides a method of obtaining an onset hastened analgesic and/or anti-pyretic response comprising the administration of a fast-release homogeneous compressed tablet composition comprising:-
i) 1-50% by weight flurbiprofen or a pharmaceutically acceptable salt thereof; and
ii) 50-99% by weight compressible carrier material comprising a disintegrant and a compressible component selected from a sugar component, a starch component and an alkaline earth metal component;
characterised in that the compressible carrier material further comprises microcrystalline cellulose present in a ratio to said compressible component of 1:4 to 4:1 parts by weight;
and further characterised in that the crushing strength of the tablet is in the range 5-15 kgf and that the disintegration time is less than 10 minutes.

The preparation of compressed tablets from formulations of the present invention is illustrated by the following Examples. In the Examples, flurbiprofen and the sodium salt thereof is available from Knoll Pharmaceuticals, Nottingham, GB; microcrystalline cellulose is available from the FMC Corporation, Brussels, BE under the trade name Avicel PH101; croscarmellose sodium is available from the FMC Corporation, Brussels, BE under the trade name Ac-Di-Sol; colloidal silicon dioxide is available from Degussa, Frankfurt, DE under the trade name Aerosil 300; polyvinylpyrrolidone is available from ISP, Guildford, GB under the trade name Plasdone K29-32; sodium starch glycollate is available from Edward Mendell, Reigate, GB under the trade name Explotab and tricalcium phosphate is available from Albright and Wilson, Warley, GB.

### A. Method of Preparation of Tablets in the Illustrative Examples

The tablets were prepared by feeding the flurbiprofen or sodium salt thereof, the microcrystalline cellulose, the compressible component (lactose/maize starch/tricalcium phosphate) and the disintegrant (croscarmellose sodium) to a conventional pre-granulating machine to form a homogenous powder mixture. A granulating solution of polyvinylpyrrolidone dissolved in isopropyl alcohol was fed into the powder mixture and the mixture blended until a satisfactory granular product was formed. The granules were dried and then sieved to a median particle size of 522 µm to remove the larger granules. Magnesium stearate and colloidal silicon dioxide were blended with the flurbiprofen granules for 3-5 minutes. The mixture was then fed to a conventional tabletting machine and formed into tablets at compression forces in the range 100-200 MPa.

### B. Measurement of the Properties of the Tablets Prepared in the Examples

Using a compaction simulator, the tablets were compressed at a range of compression forces. Crushing strength (kgf) and disintegration time (seconds) measurements were taken and best fit curves were drawn plotting crushing strength vs compaction pressure and disintegration time vs compaction pressure.

For Examples 1-6, the figures given represent the whole range of crushing strengths and whole range of disintegration times for tablets produced at all compression forces in the range 100 to 150 MPa. For Examples 7-12 the crushing strength values and disintegration times given are read off from the best-fit curves at compression forces of 150 MPa and 200 MPa respectively.

### 1. Crushing Strength (kgf)

The crushing strength is a measure of the hardness of a tablet. It was measured by recording the diametrical crushing strength when the tablet was broken by the motorised jaws of a Schleuniger crushing strength tester. The crushing strengths of five tablets prepared with each Example formulation is measured.

### 2. Disintegration Time (Seconds)

The disintegration time was measured using the disintegration method described in the European Pharmacopoeia 1986, Ref V.5.1.1 (updated 1995) using tap water (pH approximately 7) as the liquid. The method provides the time by which six tablets prepared with each Example formulation had all disintegrated.

### 3. Dissolution Time (Seconds)

The dissolution of tablets made according to Examples 7-12 and the comparative Example was measured as set out in USP 23 (1995 Edition) Dissolution Apparatus 2 (page 690). The mean % dissolved after 10, 20, 30, 45 and 60 minutes for each of these tablets is given.

### C. Example Tablets and Properties Thereof

% are given as % weight of the tablet.

Tablets were prepared from the ingredients listed in each Example as described under Heading A above (Method of Preparation of Tablets in the Illustrative Examples) and the crushing strength, disintegration time and dissolution time were measured as described under Heading B above (Measurement of the Properties of the Tablets Prepared in the Examples).

### Examples 1-2

| Ingredients | Example 1 | Example 2 |
|---|---|---|
| **Content of drug per tablet (mg)** | 5.0 mg | 6.25 |
| **Tablet weight (mg)** | 170 mg | 170 mg |
| | | |
| Racemic flurbiprofen | 2.9% | 3.6% |
| Microcrystalline cellulose | 37.9% | 37.5% |
| Lactose | 47.4% | 47.1% |
| Croscarmellose sodium | 5.0% | 5.0% |
| Polyvinylpyrrolidone | 5.0% | 5.0% |
| Colloidal silicon dioxide | 1.0% | 1.0% |
| Magnesium stearate | 0.8% | 0.8% |

| **Properties of Tablet** | **Example 1** |
|---|---|
| Crushing Strength Range (kgf) | 7-9 |
| Disintegration Time (s) | 60-140 |

### Examples 3-4

| Ingredients | Example 3 | Example 4 |
|---|---|---|
| **Content of drug per tablet (mg)** | 25 mg | 50 mg |
| **Core tablet weight (mg)** | 170 mg | 260 mg |
| | | |
| Racemic flurbiprofen | 14.7% | 19.2% |
| Microcrystalline cellulose | 32.1% | 30.6% |
| Lactose | 41.5% | 37.4% |
| Croscarmellose sodium | 5.0% | 6.0% |
| Polyvinylpyrrolidone | 5.0% | 5.0% |
| Colloidal silicon dioxide | 1.0% | 1.0% |
| Magnesium stearate | 0.7% | 0.8% |

| **Properties of Tablet** | **Example 3** | **Example 4** |
|---|---|---|
| Crushing Strength Range (kgf) | 5-8 | 5-10 |
| Disintegration time (s) | 60-120 | 70-140 |

Examples 1-3 were coated with a film coating applied according to conventional tablet film coating technology comprising:-
Opaspray white M-1-7111B (1.1 mg per tablet), French Chalk (0.7 mg per tablet), Hydroxypropylmethylcellulose 2910 USP 606 (3.3 mg per tablet).

Examples 1-3 having a film coating also had a crushing strength in the range 5-15 kgf and a disintegration time of less than 10 minutes.

### Examples 5-6

| Ingredients | Example 5 | Example 6 |
|---|---|---|
| **Content of drug per tablet (mg)** | 50 mg | 100 mg |
| **Tablet weight (mg)** | 500 mg | 500 mg |
| | | |
| Racemic flurbiprofen | 10.0% | 20.0% |
| Microcrystalline cellulose | 33.3% | 27.5% |
| Lactose | 45.0% | 40.8% |
| Croscarmellose sodium | 5.0% | 5.0% |
| Polyvinylpyrrolidone | 5.0% | 5.0% |
| Colloidal silicon dioxide | 1.0% | 1.0% |
| Magnesium stearate | 0.7% | 0.7% |

| **Properties of Tablet** | **Example 5** | **Example 6** |
|---|---|---|
| Crushing Strength Range (kgf) | 16-21 | - (No data available) |
| Disintegration time (s) | 100-240 | 70-140 |

### Examples 7-8

| Ingredients | Example 7 | Example 8 |
|---|---|---|
| **Content of drug per tablet (mg)** | 25 mg | 25 mg |
| **Tablet weight (mg)** | 170 mg | 170 mg |
| | | |
| Racemic flurbiprofen | 14.7% | 14.7% |
| Microcrystalline cellulose | 32.1% | 58.9% |
| Polyvinylpyrrolidone | 5.0% | 5.0% |
| Croscarmellose sodium | 5.0% | 5.0% |
| Maize Starch | 41.5% | 14.7% |
| Magnesium stearate | 0.7% | 0.7% |
| Colloidal silicon dioxide | 1.0% | 1.0% |

| **Properties of Tablet** | **Example 7** | |
|---|---|---|
| Compression force (MPa) | 150 | 200 |
| Crushing Strength (kgf) | 7.8 | 9.8 |
| Disintegration Time (s) | 47 | 64 |

| **Properties of Tablet** | **Example 8** | |
|---|---|---|
| Compression force (MPa) | 150 | 200 |
| Crushing Strength (kgf) | 7 | 8.5 |
| Disintegration Time (s) | 46 | 74 |

### Examples 9-10

| Ingredients | Example 9 | Example 10 |
|---|---|---|
| **Content of drug per tablet (mg)** | 25 mg | 25 mg |
| **Tablet weight (mg)** | 170 mg | 170 mg |
| | | |
| Racemic flurbiprofen | 14.7% | 14.7% |
| Microcrystalline cellulose | 32.1% | 32.1% |
| Polyvinylpyrrolidone | 5.0% | 5.0% |
| Croscarmellose sodium | 8.0% | - |
| Sodium starch glycollate | - | 8.0% |
| Lactose | 38.5% | 38.5% |
| Magnesium stearate | 0.7% | 0.7% |
| Colloidal silicon dioxide | 1.0% | 1.0% |

| **Properties of Tablet** | **Example 9** | |
|---|---|---|
| Compression force (MPa) | 150 | 200 |
| Crushing Strength (kgf) | 8.1 | 9.6 |
| Disintegration Time (s) | 132 | 185 |

| **Properties of Tablet** | **Example 10** | |
|---|---|---|
| Compression force (MPa) | 150 | 200 |
| Crushing Strength (kgf) | 8.4 | 10.1 |
| Disintegration Time (s) | 155 | 254 |

### Examples 11-12

| Ingredients | Example 11 | Example 12 |
|---|---|---|
| **Content of drug per tablet (mg)** | 25 mg | 31 mg |
| **Tablet weight (mg)** | 170 mg | 176 mg |
| | | |
| Racemic flurbiprofen | 14.7% | - |
| Racemic flurbiprofen (Na salt) | - | 17.6% |
| Microcrystalline cellulose | 36.8% | 31.0% |
| Polyvinylpyrrolidone | 5.0% | 4.8% |
| Croscarmellose sodium | 5.0% | 4.8% |
| Tricalcium phosphate | 36.8% | - |
| Lactose | - | 40.1% |
| Magnesium stearate | 0.7% | 0.7% |
| Colloidal silicon dioxide | 1.0% | 1.0% |

| **Properties of Tablet** | **Example 11** | |
|---|---|---|
| Compression force (MPa) | 150 | 200 |
| Crushing Strength (kgf) | 6.8 | 8.5 |
| Disintegration Time (s) | 90 | 153 |

| **Properties of Tablet** | **Example 12** | |
|---|---|---|
| Compression force (MPa) | 150 | 200 |
| Crushing Strength (kgf) | 7.3 | 9.5 |
| Disintegration Time (s) | 160 | 259 |

It can be seen that all the illustrative Examples have good values for the crushing strength and also relatively low disintegration times.

### Dissolution Results of Examples 7-12 and the Comparative Example

The dissolution method is described under heading B above (Measurement of the Properties of the Tablets Prepared in the Examples: Dissolution Time).

The commercially available comparative Example (50mg Cebutid tablet) (uncoated) contains as the main ingredients racemic flurbiprofen (50mg) together with lactose and maize starch as the compressible carrier, polyvinylpyrrolidone as the binder, magnesium stearate and stearic acid as tablet lubricants (reference Dictionnaire Vidal 1997) - total tablet weight = 100mg. In the comparative Example the flurbiprofen tablet was prepared in a similar manner to that of the illustrative Examples by preparing a pre-granulated powder mixture containing the flurbiprofen, the maize starch and the lactose. A granular product was formed by granulating with polyvinylpyrrolidone in a solvent. The tabletting excipients comprising magnesium stearate and stearic acid were blended with the flurbiprofen granules before compressing into tablets.

The % dissolved from each of the Example formulations 7-12 and the comparative Example is shown in the table below. % greater than 100% fall within the 5% calibration accuracy of the apparatus. Figures greater than 95% show that substantially all of the drug has dissolved. These results show very good dissolution of tablets according to the present invention.

| **Example** | **10 min** | **20 min** | **30 min** | **45 min** | **60 min** |
|---|---|---|---|---|---|
| 7 | 96.0% | 104.4% | 105.7% | 106.4% | 106.7% |
| 8 | 96.9% | 101.4% | 101.9% | 101.9 % | 101.6% |
| 9 | 100.5% | 104.3% | 104.4% | 104.4% | 104.4% |
| 10 | 95.0% | 103.3% | 103.4% | 103.3% | 103.5% |
| 11 | 78.3% | 86.5% | 88.7% | 91.1% | 93.1% |
| 12 | 89.7% | 95.2% | 95.4% | 95.3% | 95.5% |
| Comparative Example | 17.0% | 58.1% | 91.0% | 99.3% | 100.4% |

### D. Drawings

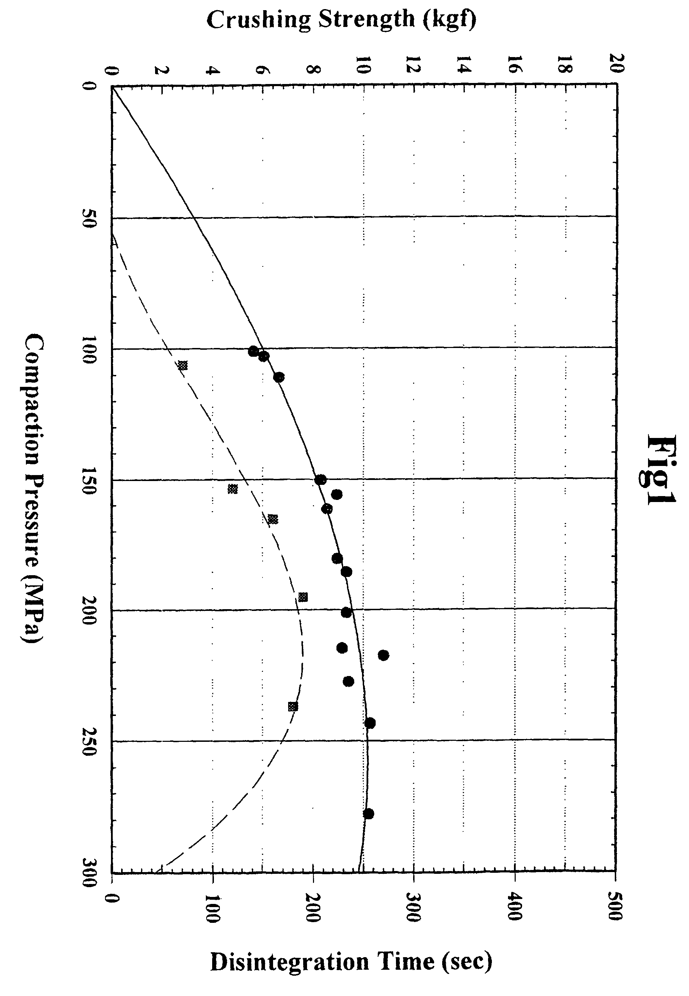

In the drawings:-
Fig 1 represents a graph of
   (1) crushing strength (kgf) (shown by the continuous line) versus compaction pressure (MPa), and
   (2) disintegration time (seconds) (dashed line) versus compaction pressure (MPa)
   for round tablets (7 mm diameter) prepared as described in Example 9.
   It can be seen that the crushing strength curve (continuous line) shows an advantageous shallow gradient. The mean crushing strength of the tablets is in excess of 6 kgf above a compaction pressure of 100MPa. The disintegration curve (dashed line) shows the mean disintegration times to be less than 200 seconds at all compaction pressures.

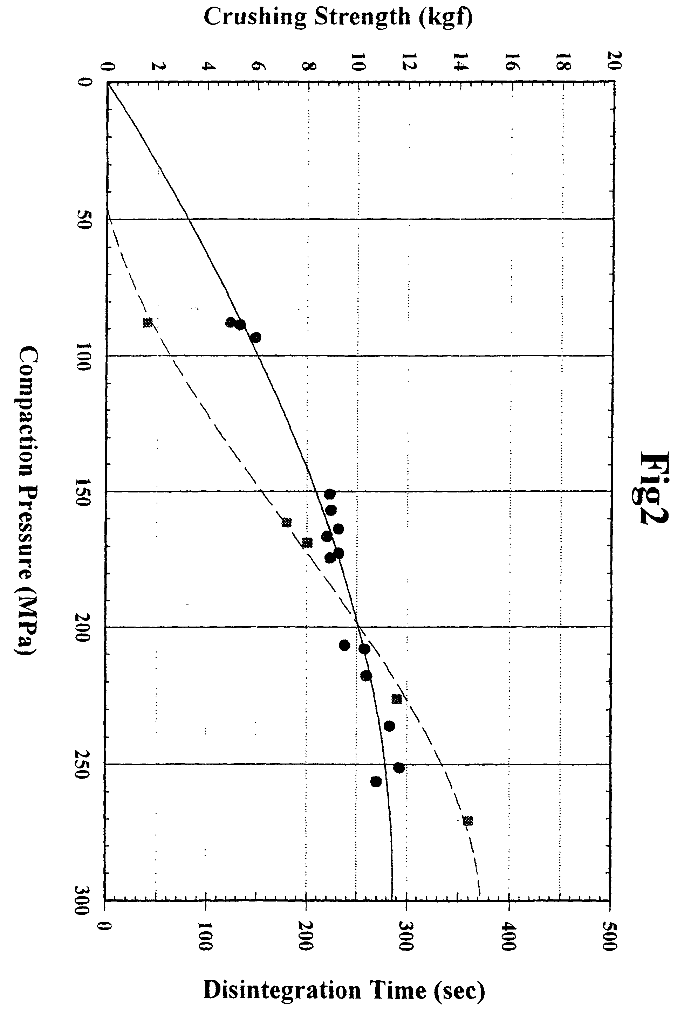

Fig 2 represents a graph of
   (1) crushing strength (kgf) (shown by the continuous line) versus compaction pressure (MPa), and
   (2) disintegration time (seconds) (shown by the dotted line) versus compaction pressure (MPa)
   for round tablets (7 mm diameter) prepared as described in Example 10.
   It can be seen that the crushing strength curve (continuous line) shows an advantageous shallow gradient. The mean crushing strength of the tablets is in excess of 6 kgf above a compaction pressure of 100MPa. The disintegration curve (dashed line) shows the mean disintegration times to be less than 300 seconds up to a compaction pressure of 225MPa.

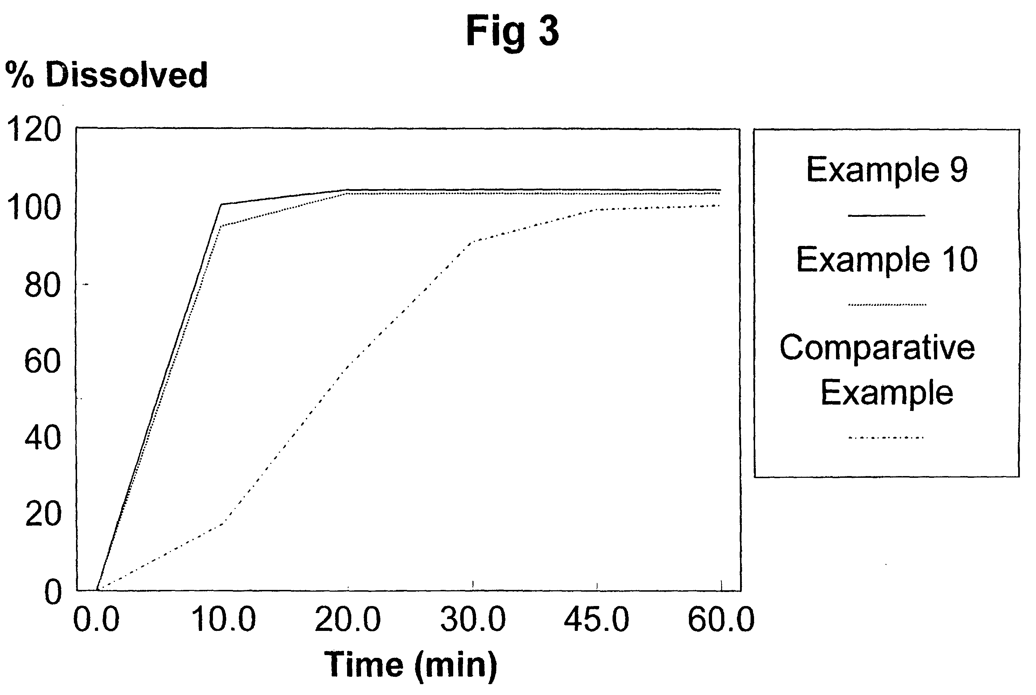

Fig 3 (Comparative Example) represents a graph plotting % of drug dissolved from
   (a) tablets according to the present invention prepared as described in Examples 9 and 10 and
   (b) a commercially available flurbiprofen tablet (Cebutid) (uncoated) containing 50mg racemic ibuprofen prepared as described in the comparative Example above.
   It can be seen that substantially all of the flurbiprofen is dissolved from illustrative Examples 9 and 10 within 10 minutes, whereas the comparative Examples does not approach these values until at least 45 minutes.

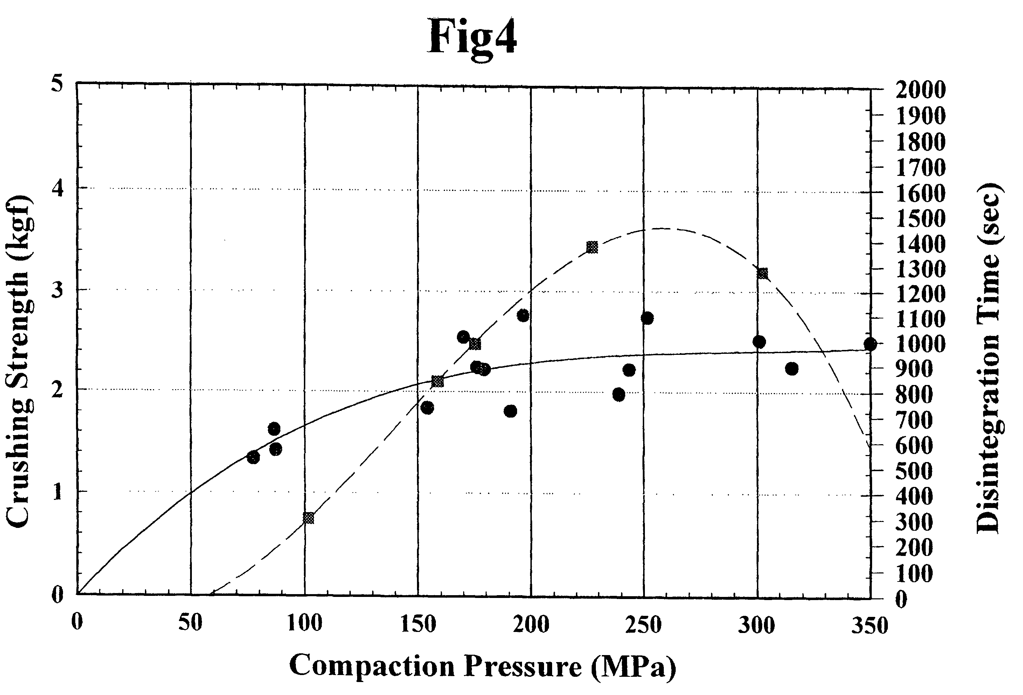

Fig 4 (Comparative Example) represents a graph of
   (1) crushing strength (kgf) (shown by the continuous line) versus compaction pressure (MPa)
      and
   (2) disintegration time (seconds) (shown by the dashed line) versus compaction pressure (MPa)
   for round tablets (6.5 mm diameter) of a commercially available flurbiprofen tablet (Cebutid) (uncoated) containing 50mg racemic flurbiprofen prepared as described in the comparative Example above.
   It can be seen that the mean crushing strength curve (continuous line) is less than 2.5 kgf even at compaction pressures of greater than 200MPa. The disintegration curve (dashed line) shows the mean disintegration times to be greater than 800 seconds if crushing strength in excess of 2kgf is required.

## Claims

1. A fast-release homogeneous compressed tablet composition comprising:-
i) 1-50% by weight flurbiprofen or a pharmaceutically acceptable salt thereof; and
ii) 50-99% by weight compressible carrier material comprising a disintegrant and a compressible component selected from a sugar component, a starch component and an alkaline earth metal component;
**characterised in that** the compressible carrier material further comprises microcrystalline cellulose present in a ratio to said compressible component of 1:4 to 4:1 parts by weight;
and further **characterised in that** the crushing strength of the tablet as measured by a Schleuniger hardness meter in the range 5-15 Kgf and that the disintegration time measured according to the European Pharmacopoeia, 1986, Ref V.5.1.1(updated 1997) is less than 10 minutes.

2. A compressed tablet composition according to claim 1 **characterised by** comprising 2-30% by weight flurbiprofen or a soluble salt thereof.

3. A compressed tablet composition according to either one of claims 1 and 2 **characterised by** comprising 2.5-20% by weight racemic flurbiprofen.

4. A compressed tablet according to any one of claims 1 to 3 **characterised in that** the ratio of microcrystalline cellulose to said compressible component is in the range 4:1 to 1:2.

5. A compressed tablet according to any one of claims 1 to 4 **characterised in that** the ratio of microcrystalline cellulose to said compressible component is in the range 1:1 to 1:2.

6. A compressed tablet according to any one of claims 1 to 5 **characterised in that** the crushing strength of the tablet is in the range 5-10 kgf when compressed at a compression force in the range 100-150MPa.

7. A compressed tablet according to any one of claims 1 to 6 **characterised in that** the disintegration time of the tablet is less than 5 minutes when compressed at a compression force in the range 100-150MPa.

8. A compressed tablet composition according to any one of claims 1 to 7 **characterised in that** the compressible component is present to an extent of 60-95% of the composition and comprises one or more components selected from lactose, sucrose, dextrose, dextrin, cyclodextrin, maltodextrin, mannitol, sorbitol, xylitol, maize starch, potato starch, wheat starch, pre-gelatinised starch, calcium sulphate, calcium carbonate, calcium phosphate compounds, magnesium sulphate, magnesium carbonate, magnesium oxide and magnesium hydroxide.

9. A compressed tablet according to any one of claims 1 to 8 **characterised in that** the compressible component comprises lactose, tricalcium phosphate and maize starch or a mixture thereof.

10. A compressed tablet according to any one of claims 1 to 9 **characterised in that** the disintegrant is selected from croscarmellose sodium and sodium starch glycolate.

11. A compressed tablet according to any one of claims 1 to 10 **characterised in that** the compressible carrier material consists essentially of a disintegrant, a compressible component selected from a sugar component, a starch component and an alkaline earth metal component and microcrystalline cellulose.

12. A compressed tablet according to any one of claims 1 to 11 **characterised in that** the compressible carrier material comprises:-
(a) 5-10% by weight disintegrant
(b) 10-50% by weight compressible component
(c) 25-60% by weight microcrystalline cellulose the sum of (a), (b) and (c) being 100% by weight.

13. A compressed tablet according to any one of claims 1 to 12 **characterised in that** the compressible carrier material consists essentially of croscarmellose sodium, microcrystalline cellulose and lactose.

14. A process to prepare a tablet composition as claimed in any one of claims 1-13 comprising the steps of (a) combining flurbiprofen with the components of the compressible carrier material to form a homogeneous mixture and (b) compressing the mixture into tablets.

15. A process according to claim 14 **characterised in that**:-
(a) the flurbiprofen is formed into a granule solely with the compressible carrier material in the presence of a an inert solvent;
(b) the granules are dried and a tablet lubricant optionally with a flow aid is combined with the granules; and
(c) the mixture is fed to a tabletting machine and compressed into tablets at a compression force in the range 100-140MPa.

16. A process according to claim 14 wherein all the ingredients are mixed together as a dry powder blend followed by direct compression into tablets.

17. Use of a compressed tablet as claimed in any one of claims 1 to 13 for the preparation of a medicament for treating pain, fever and inflammation.

## Patentansprüche

1. Schnellfreisetzende, homogene, komprimierte Tablettenzusammensetzung, enthaltend:
i) 1-50 Gew.-% Flurbiprofen oder ein pharmazeutisch unbedenkliches Salz davon, und
ii) 50-99 Gew.-% eines komprimierbaren Trägermaterials, enthaltend ein Sprengmittel und eine komprimierbare Komponente ausgewählt aus einer Zuckerkomponente, einer Stärkekomponente und einer Erdalkalimetallkomponente,
**dadurch gekennzeichnet, daß** das komprimierbare Trägermaterial weiterhin mikrokristalline Cellulose enthält, die zur komprimierbaren Komponente in einem Verhältnis von 1:4 bis 4:1 Gewichtsteilen vorliegt,
und weiterhin **dadurch gekennzeichnet, daß** die mit einem Schleuniger-Härtemesser gemessene Druckfestigkeit der Tablette im Bereich von 5-15 kgf und die gemäß der European Pharmacopoeia, 1986, Ref. V.5.1.1 (überarbeitet 1997), gemessene Zerfallszeit weniger als 10 Minuten beträgt.

2. Komprimierte Tablettenzusammensetzung nach Anspruch 1, **dadurch gekennzeichnet, daß** sie 2-30 Gew.-% Flurbiprofen oder eines löslichen Salzes davon enthält.

3. Komprimierte Tablettenzusammensetzung nach Anspruch 1 oder 2, **dadurch gekennzeichnet, daß** sie 2,5-20 Gew.-% racemisches Flurbiprofen enthält.

4. Komprimierte Tablette nach einem der Ansprüche 1 bis 3, **dadurch gekennzeichnet, daß** das Verhältnis von mikrokristalliner Cellulose zu komprimierbarer Komponente im Bereich von 4:1 bis 1:2 liegt.

5. Komprimierte Tablette nach einem der Ansprüche 1 bis 4, **dadurch gekennzeichnet, daß** das Verhältnis von mikrokristalliner Cellulose zu komprimierbarer Komponente im Bereich von 1:1 bis 1:2 liegt.

6. Komprimierte Tablette nach einem der Ansprüche 1 bis 5, **dadurch gekennzeichnet, daß** die Druckfestigkeit der Tablette im Bereich von 5-10 kgf liegt, wenn mit einer Kompressionskraft im Bereich von 100-150 MPa komprimiert wird.

7. Komprimierte Tablette nach einem der Ansprüche 1 bis 6, **dadurch gekennzeichnet, daß** die Zerfallszeit der Tablette weniger als 5 Minuten beträgt, wenn mit einer Kompressionskraft im Bereich von 100-150 MPa komprimiert wird.

8. Komprimierte Tablettenzusammensetzung nach einem der Ansprüche 1 bis 7, **dadurch gekennzeichnet, daß** die komprimierbare Komponente in einem Anteil von 60-95% der Zusammensetzung vorliegt und eine oder mehrere Komponenten ausgewählt aus Laktose, Saccharose, Dextrose, Dextrin, Cyclodextrin, Maltodextrin, Mannit, Sorbit, Xylit, Maisstärke, Kartoffelstärke, Weizenstärke, vorgelatinierter Stärke, Calciumsulfat, Calciumcarbonat, Caiciumphosphatverbindungen, Magnesiumsulfat, Magnesiumcarbonat, Magnesiumoxid und Magnesiumhydroxid enthält.

9. Komprimierte Tablette nach einem der Ansprüche 1 bis 8, **dadurch gekennzeichnet, daß** die komprimierbare Komponente Laktose, Tricalciumphosphat und Maisstärke oder eine Mischung davon enthält.

10. Komprimierte Tablette nach einem der Ansprüche 1 bis 9, **dadurch gekennzeichnet, daß** das Sprengmittel aus Croscarmellose-Natrium und Natrium-Stärkeglykolat ausgewählt ist.

11. Komprimierte Tablette nach einem der Ansprüche 1 bis 10, **dadurch gekennzeichnet, daß** das komprimierbare Trägermaterial im wesentlichen aus einem Sprengmittel, einer komprimierbaren Komponente ausgewählt aus einer Zuckerkomponente, einer Stärkekomponente und einer Erdalkalimetallkomponente und mikrokristalliner Cellulose besteht.

12. Komprimierte Tablette nach einem der Ansprüche 1 bis 11, **dadurch gekennzeichnet, daß** das komprimierbare Trägermaterial
(a) 5-10 Gew.-% Sprengmittel
(b) 10-50 Gew.-% komprimierbare Komponente
(c) 25-60 Gew.-% mikrokristalline Cellulose
enthält, wobei die Summe von (a), (b) und (c) 100 Gew.-% beträgt.

13. Komprimierte Tablette nach einem der Ansprüche 1 bis 12, **dadurch gekennzeichnet, daß** das komprimierbare Trägermaterial im wesentlichen aus Croscarmellose-Natrium, mikrokristalliner Cellulose und Laktose besteht.

14. Verfahren zur Herstellung einer Tablettenkomposition nach einem der Ansprüche 1-13, bei dem man (a) Flurbiprofen mit den Komponenten des komprimierbaren Trägermaterials zu einer homogenen Mischung kombiniert und (b) die Mischung zu Tabletten verpreßt.

15. Verfahren nach Anspruch 14, **dadurch gekennzeichnet, daß** man
(a) das Flurbiprofen alleine mit dem komprimierbaren Trägermaterial in Gegenwart eines inerten Lösungsmittels zu einem Granulat formt,
(b) das Granulat trocknet und ein Tablettengleitmittel, gegebenenfalls mit einer Fließhilfe, mit dem Granulat kombiniert und
(c) die Mischung einer Tablettiermaschine zuführt und bei einem Kompressionsdruck im Bereich von 100-140 MPa zu Tabletten komprimiert.

16. Verfahren nach Anspruch 14, bei dem man die Inhaltsstoffe als Trockenpulvermischung miteinander vermischt und dann direkt zu Tabletten komprimiert.

17. Verwendung einer komprimierten Tablette nach einem der Ansprüche 1 bis 13 zur Herstellung eines Medikaments zur Behandlung von Schmerzen, Fieber und Entzündungen.

## Revendications

1. Composition de comprimé homogène à libération rapide comprenant :
i) de 1 à 50 % en poids de flurbiprofène ou un sel pharmaceutiquement acceptable de celui-ci ; et
ii) de 50 à 99 % en poids de matière support comprimable comprenant un agent délitant et un composant comprimable choisi parmi un composant sucre, un composant amidon et un composant métal alcalino-terreux ;
**caractérisée en ce que** la matière support comprimable comprend de plus de la cellulose microcristalline présente dans un rapport audit composant comprimable de 1:4 à 4:1 parties en poids ; et **caractérisée de plus en ce que** la résistance du comprimé à l'écrasement telle qu'elle est mesurée par un duromètre de Schleuniger est dans la gamme de 5 à 15 Kgf et **en ce que** le temps de délitement mesuré selon la Pharmacopée Européenne 1986, réf. V.5.1.1 (mise à jour 1997) est inférieur à 10 minutes.

2. Composition de comprimé selon la revendication 1, **caractérisée en ce qu'**elle comprend de 2 à 30 % en poids de flurbiprofène ou un sel soluble de celui-ci .

3. Composition de comprimé selon la revendication 1 ou la revendication 2, **caractérisée en ce qu'**elle comprend de 2,5 à 20 % en poids de flurbiprofène racémique.

4. Comprimé selon l'une quelconque des revendications 1 à 3, **caractérisé en ce que** le rapport de cellulose microcristalline audit composant comprimable est dans la gamme de 4:1 à 1:2.

5. Comprimé selon l'une quelconque des revendications 1 à 4, **caractérisé en ce que** le rapport de la cellulose microcristalline audit composant comprimable est dans la gamme de 1:1 à 1:2.

6. Comprimé selon l'une quelconque des revendications 1 à 5, **caractérisé en ce que** la résistance du comprimé à l'écrasement est dans la gamme de 5 à 10 Kgf lorsque la compression est réalisée à une force de compression dans la gamme de 100 à 150 MPa.

7. Comprimé selon l'une quelconque des revendications 1 à 6, **caractérisé en ce que** le temps de délitement du comprimé est inférieur à 5 minutes lorsque la compression est réalisée à une force de compression dans la gamme de 100 à 150 MPa.

8. Composition de comprimé selon l'une quelconque des revendications 1 à 7, **caractérisée en ce que** le composant comprimable est présent dans un taux de 60 à 95 % de la composition et comprend un ou plusieurs composants choisis parmi le lactose, le saccharose, le dextrose, la dextrine, la cyclodextrine, la maltodextrine, le mannitol, le sorbitol, le xylitol, l'amidon de maïs, l'amidon de pomme de terre, l'amidon de blé, l'amidon pré-gélatinisé, le sulfate de calcium, le carbonate de calcium, les composés de phosphate de calcium, le sulfate de magnésium, le carbonate de magnésium, l'oxyde de magnésium et l'hydroxyde de magnésium.

9. Comprimé selon l'une quelconque des revendications 1 à 8, **caractérisé en ce que** le composant comprimable comprend le lactose, le phosphate tricalcique et l'amidon de maïs ou un mélange de ceux-ci.

10. Comprimé selon l'une quelconque des revendications 1 à 9, **caractérisé en ce que** l'agent délitant est choisi parmi le croscarmellose sodique et le glycolate d'amidon sodique.

11. Comprimé selon l'une quelconque des revendications 1 à 10, **caractérisé en ce que** la matière support comprimable est constituée essentiellement d'un agent délitant, d'un composant comprimable choisi parmi un composant sucre, un composant amidon et un composant métal alcalino-terreux et de la cellulose microcristalline.

12. Comprimé selon l'une quelconque des revendications 1 à 11, **caractérisé en ce que** la matière support comprimable comprend :
(a) de 5 à 10 % en poids d'agent délitant
(b) de 10 à 50 % en poids de composant comprimable
(c) de 25 à 60 % en poids de cellulose microcristalline,
la somme de (a), (b) et (c) étant de 100 % en poids.

13. Comprimé selon l'une quelconque des revendications 1 à 12, **caractérisé en ce que** la matière support comprimable est constituée essentiellement de croscarmellose sodique, de cellulose microcristalline et de lactose.

14. Procédé de préparation d'une composition de comprimé selon l'une quelconque des revendications 1 à 13, comprenant les étapes de (a) combiner le flurbiprofène avec les composants de la matière support comprimable pour former un mélange homogène et (b) mettre le mélange en comprimés.

15. Procédé selon la revendication 14, **caractérisé en ce que** :
(a) le flurbiprofène est formé en un granulé uniquement avec la matière support comprimable en présence d'un solvant inerte ;
(b) les granulés sont séchés et un lubrifiant de comprimé, éventuellement avec un agent d'écoulement, est combiné avec les granulés ; et
(c) le mélange est apporté dans une machine à comprimé et mis en comprimés à une force de compression dans la gamme de 100 à 140 MPa.

16. Procédé selon la revendication 14, **caractérisé en ce que** tous les ingrédients sont mélangés ensemble sous forme de mélange de poudre sèche suivi de compression directe en comprimés.

17. Utilisation d'un comprimé selon l'une quelconque des revendications 1 à 13, pour la préparation de médicament destiné au traitement de la douleur, de la fièvre et de l'inflammation.
